# EUROPEAN PATENT APPLICATION

(11) **EP 2 030 973 A1**
(43) Date of publication of application: **04.03.2009**
(21) Application number: 07115432.2
(22) Date of filing: 31.08.2007
(51) Int. Cl.: C07D 401/12

(54) **Process for preparing 2-sulfinyl-1H-benzimidazoles**

(71) Applicant: KRKA, tovarna zdravil, d.d., Novo mesto, 8501 Novo mesto (SI)
(72) Inventor: Iskra, Jernei, 1000, Ljubljana (SI); Stavber, Stojan, 1295, Ivancana Gorica (SI); Kotar Jordan, Berta, 8311, Kostanjevica na Krki (SI); Ruzic, Milos, 3000, Celje (SI); Smodis, Janez, 8000, Novo mesto (SI); Zupet, Rok, 1211, Ljubljana (SI)
(74) Representative: UEXKÜLL & STOLBERG

(57) **Abstract**

The present invention relates to a process for preparing 2-(2-pyridinylmethylsulfinyl)-1*H*-benzimidazoles by oxidizing a thioether precursor in the presence of trifluoroethanol.

## Description

### FIELD OF THE INVENTION

The present invention relates to a process for preparing 2-(2-pyridinylmethylsulfinyl)-1*H*-benzimidazoles such as pantoprazole, lansoprazole, omeprazole and rabeprazole.

### BACKGROUND OF THE INVENTION

Substituted 2-(2-pyridinylmethylsulfinyl)-1*H*-benzimidazole compounds are well-known gastric proton pump inhibitors. These compounds can inhibit gastric acid secretion and are used as an antiulcer agent. They can be represented by the following generic formula, wherein R¹ to R⁴ have the same meanings as described below:

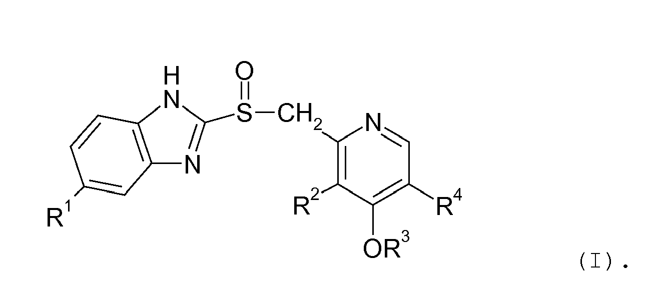

Examples for such benzimidazole compounds include 2-[[[3-methyl-4-(2,2,2-trifluoroethoxy)-2-pyridinyl]methyl]sulfinyl]-1H-benzimidazole (lansoprazole), 5-(difluoromethoxy)-2-[[[3,4-dimethoxy-2-pyridinyl]methyl]sulfinyl]-1*H*-benzimidazole (pantoprazole), 5-methoxy-2-[[[4-methoxy-3,5-dimethyl-2-pyridinyl]methyl]sulfinyl]-1*H*-benzimidazole (omeprazole) and 2-[[[4-(3-methoxypropoxy)-3-methyl-2-pyridinyl]methyl]sulfinyl]-1*H*-benzimidazole (rabeprazole).

Processes for preparing these benzimidazoles are known. Several methods involve the use of a precursor having a thioether group which is subjected to an oxidation.

EP 0 174 726 B1 discloses the use of a peracid such as m-chloroperbenzoic acid, sodium bromite, sodium hypochlorite or hydrogen peroxide as an oxidizing agent. The oxidation is carried out in halogenated hydrocarbons, amides, alcohols or mixtures thereof.

EP 0 302 720 A1 describes a process for preparing 2-sulfinylbenzimidazoles using hydrogen peroxide in the presence of a vanadium compound such as vanadium pentoxide, sodium metavanadate or vanadium acetylacetonate.

According to WO 02/062786 A1, the oxidation of the thioether precursor is performed by using tert-butyl hydroperoxide (TBHP) in the presence of vanadium pentoxide, sodium metavanadate or vanadium acetylacetonate. Preferably, the oxidation is carried out in toluene or isopropanol.

WO 2004/011455 A1 discloses a process for preparing lansoprazole using tert-butyl hydroperoxide (TBHP) in the presence of vanadium oxytrichloride as catalyst wherein the reaction is carried out in a solvent such as a C₁- to C₅-alcohol, decane, nonane, toluene or a mixture with water. Moreover, the reaction is preferably performed in the presence of a weak base.

WO 03/008406 A1 refers to a process for preparing benzimidazole-type compounds by reacting a corresponding precursor with an oxidizing agent in a suitable solvent, extracting the sulphone by-product and isolating the product. Preferably, m-chloroperbenzoic acid is used as oxidizing agent.

EP 0 997 461 A1 describes the oxidation of a thioether benzimidazole to the corresponding sulfoxide compound by using N-halosuccinimide, 1,3-dihalo-5,5-dimethylhydantoin or dichloroisocyanurate in the presence of base. Alternatively, a perborate compound in the presence of acid anhydride or a metal catalyst can be used as oxidizing agent.

WO 01/21617 A1 discloses a process for preparing lansoprazole by using hydrogen peroxide in the presence of a rhenium catalyst. Preferably, methyltrioxorhenium is used as catalyst. The oxidation is performed in ethanol. The selectivity obtained in the oxidation step is reported to be acceptable, if the reaction is carried out at a temperature of -20 to -30°C and the amount of the rhenium compound is 1 to 5 mole% relative to the starting material. At higher rection temperatures and lower catalyst concentrations the formation of impurities increases.

Finally, WO 2004/056803 A1 describes a process for preparing sulfinyl derivatives by using hydrogen peroxide as oxidizing agent in the presence of a rhenium compound. In contrast to WO 01/21617 A1, this document teaches to use the rhenium catalyst in an amount of 0.01 to 0.5 mole% with respect to the sulfide starting compound and to keep the reaction temperature from 0°C to room temperature. Linear or branched C₁-C₆-alcohols, ketones, esters, ethers or amides can be used alone or in admixture with water as a solvent during the oxidation step. Preferably, methanol is used. At a reaction temperature of 5°C, a catalyst concentration of 0.1 mole% and a reaction time of 4 hours, lansoprazole is formed with a yield of 75%.

The processes according to the prior art for preparing substituted 2-(2-pyridinylmethylsulfinyl)-1*H*-benzimidazole compounds, however, still suffer from a low yield of the produced benzimidazole, a high content of impurities of the benzimidazole and/or the use of ineconomic reaction conditions. This may be due to the fact that in particular at higher rection temperatures the selectivity in the oxidation step is low and thus by-products such as the corresponding benzimidazole N-oxide and the corresponding sulphone can be formed by an oxidation of the nitrogen and an overoxidation of the sulfide, respectively.

Consequently, there is still a need for an improved process for preparing substituted 2-(2-pyridinylmethylsulfinyl)-1*H-*benzimidazole compounds avoiding the afore-mentioned drawbacks. In particular, a process would be desirable which provides an improved balance between the selectivity of the oxidation reaction, the yield of the product and the economic efficiency of the reaction conditions.

This problem is surprisingly solved by the process for preparing lansoprazole according to claims 1 to 22.

### DETAILED DESCRIPTION OF THE INVENTION

The process according to the invention for preparing a compound of formula (I) 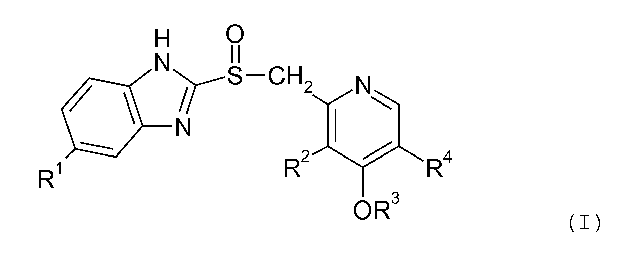 or a salt or a solvate or a hydrate thereof, wherein
- R¹: is selected from the group consisting of hydrogen, C₁-C₄-alkyl and C₁-C₄-alkoxy, wherein the C₁-C₄-alkyl and C₁-C₄-alkoxy are unsubstituted or substituted with one or more halogen,
- R²: is selected from the group consisting of hydrogen, C₁-C₄-alkyl and C₁-C₄-alkoxy, wherein the C₁-C₄-alkyl and C₁-C₄-alkoxy are unsubstituted or substituted with one or more halogen,
- R³: is C₁-C₄-alkyl which is unsubstituted or substituted with one or more halogen or one or more C₁-C₄-alkoxy, and
- R⁴: is selected from the group consisting of hydrogen and C₁-C₄-alkyl which is unsubstituted or substituted with one or more halogen,
comprises
(a) oxidizing a compound of formula (II) 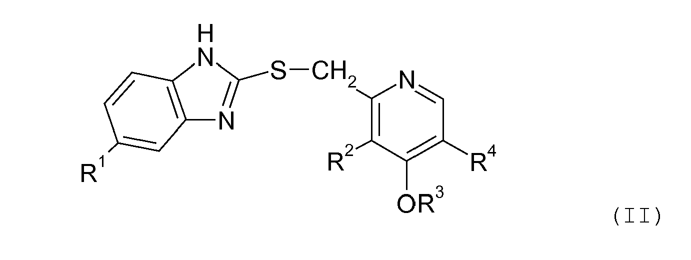 or a hydrate, solvate or salt thereof, in which R¹, R², R³ and R⁴ have the same meanings as defined above, to give the compound of formula (I), wherein the oxidizing is performed in the presence of trifluoroethanol,
(b) optionally recovering the compound of formula (I) and
(c) optionally purifying the compound of formula (I) and/or converting it into a salt or a solvate or a hydrate thereof.

It has surprisingly been found out that the presence of trifluoroethanol allows the production of substituted 2-(2-pyridinylmethylsulfinyl)-1*H*-benzimidazole compounds in high yields and leads to substituted 2-(2-pyridinylmethylsulfinyl)-1H-benzimidazole compounds which contain only small amounts of impurities.

Preferably, a compound of formula (I) is prepared in which R¹ to R⁴ have independently from each other the following meanings:
- R¹: is selected from the group consisting of hydrogen and C₁-C₄-alkoxy, which is unsubstituted or substituted with one or more halogen,
- R²: is selected from the group consisting of unsubstituted C₁-C₄-alkyl and unsubstituted C₁-C₄-alkoxy,

- R³: is C₁-C₄-alkyl which is unsubstituted or substituted with one or more halogen or one or more C₁-C₄-alkoxy, and
- R⁴: is selected from the group consisting of hydrogen and unsubstituted C₁-C₄-alkyl.

More preferably, a compound of formula (I) is prepared in which R¹ to R⁴ have independently from each other the following meanings:
- R¹: is selected from the group consisting of hydrogen, methoxy and difluoromethoxy,
- R²: is selected from the group consisting of methyl and methoxy,
- R³: is selected from the group consisting of methyl, trifluoroethyl and 3-methoxy-propyl, and
- R⁴: is selected from the group consisting of hydrogen and methyl.

Most preferably, the compound of formula (I) is pantoprazole (R¹ = -OCHF₂, R² = -OCH₃, R³ = -CH₃ and R⁴ = -H), lansoprazole (R¹ = -H, R² = -CH₃, R³ = -CH₂F₃ and R⁴ = -H), omeprazole (R¹ = - OCH₃, R² = -CH₃, R³ = -CH₃ and R⁴ = -CH₃) or rabeprazole (R¹ = - H, R² = -CH₃, R³ = -CH₂CH₂CH₂OCH₃ and R⁴ = -H).

In step (a) of the process according to the invention a compound of formula (II) or a derivative thereof is reacted. Preferably, compound (II) or a solvate or a hydrate of compound (II) is used. Commonly, a hydrate of compound (II) is used. In preferred embodiments of the process according to the invention, the definitions of R¹ to R⁴ of compound (II) correspond to the above preferred definitions of R¹ to R⁴ of compound (I). Preferably, the concentration of compound (II) in the reaction mixture ranges from 0.1 to 5.0 mol/l, more preferably 0.2 to 2.0 mol/l and most preferably 0.3 to 1.2 mol/l.

Preferably, in the process according to the invention the oxidation of the thioether group of the compound of formula (II), a hydrate, solvate or salt thereof is effected by use of hydrogen peroxide as oxidizing agent. More preferably, hydrogen peroxide is used in the form of an aqueous solution. Moreover, it is preferred that the concentration of the aqueous hydrogen peroxide solution ranges from 10 to 70 wt.-%, more preferably 20 to 50 wt.-%. Most preferably the concentration is about 30 to 35 wt.-%. It is also possible to use a source of hydrogen peroxide such as a urea adduct of hydrogen peroxide.

The amount of hydrogen peroxide used is generally about 0.5 to 3.0 equivalents, preferably 0.7 to 2.0 equivalents and most preferably 0.9 to 1.5 equivalents relative to compound (II).

Further, it is preferred that the oxidation of step (a) is performed in the presence of a metal catalyst. Preferably, the metal of the metal catalyst is selected from the group consisting of rhenium, vanadium, molybdenum, tungsten, cerium and ytterbium. More preferably, the metal catalyst is selected from the group consisting of CH₃ReO₃, C₂H₅ReO₃, Re(PPh₃)₂OCl₃, Na₂MoO₄, V₂O₅, VOCl₃, VOF₃, VO(OC₂H₅)₃, VO(1-OC₃H₇)₃, VO(2-OC₃H₇)_{3,} VO (CH₃COCHCOCH₃)₂, NaVO₃, H₂WO₄, H₄SiW₁₂O₄₀, (NH₄)₂Ce(NO₃)₆, Yb(OSO₂CF₃)₃. Most preferably, methyltrioxorhenium is used.

The amount of the metal catalyst is usually about 0.0001 to 0.1 equivalents, preferably 0.0002 to 0.01 equivalents and most preferred 0.0005 to 0.0015 equivalents relative to compound (II). If the metal catalyst is methyltrioxorhenium, it is particular preferred to use it in an amount of about 0.0005 to 0.0015 equivalents relative to compound (II) (corresponding to 0.05 to 0.15 mol.-%). The other metal compounds are usually employed at a concentration of 0.005 to 0.015 equivalents relative to compound (II) (corresponding to 0.5 to 1.5 mol.-%).

Step (a) is carried out in the presence of trifluoroethanol (CF₃CH₂OH). Further, step (a) can be performed in the presence of trifluoroethanol and an organic solvent. This organic solvent can be selected from the group consisting of methanol, ethanol, acetone, acetonitrile, C₆H₅CF₃, ethers such as THF, unpolar solvents such as dichloromethane and iso-alkanes (e.g. iso-octane), and mixtures thereof. According to a particularly preferred embodiment, step (a) is carried out in a solvent comprising trifluoroethanol. The solvent is either trifluoroethanol alone or a mixture of trifluorethanol with a second organic solvent. Preferably, this second organic solvent is selected from the group consisting of methanol, ethanol, acetone, acetonitrile, C₆H₅CF₃, ethers such as THF, unpolar solvents such as dichloromethane and iso- alkanes (e.g. *iso-*octane), and mixtures thereof. If a mixture of trifluoroethanol with a second organic solvent is used, the ratio between trifluoroethanol and said second solvent preferably ranges from 1:1 to 1:5, more preferably 1:2 to 1:4, based on the volume of the solvents. In particular, the ratio between trifluoroethanol and said second solvent is about 1:3, based on the volume of the solvents.

In a preferred embodiment of the invention, the reaction of step (a) is carried out at a temperature of -30 to 30°C, preferably -10 to 30°C and more preferably 0 to 30°C. If trifluoroethanol alone is used as solvent, the reaction temperature is preferably -20 to 20°C, more preferably -10 to 10°C and most preferably about 0°C. If a mixture of trifluoroethanol and a second organic solvent is used, the preferred reaction temperature will increase depending on the nature of said second solvent and the mixing ratio. For example, at a ratio of trifluoroethanol to second solvent of 1:3 the prefered reaction temperature generally ranges from 15 to 30°C.

It has surprisingly been found out that the use of trifluoroethanol in step (a) allows the oxidation of compound (II) into compound (I) in high yields and high selectivities. Moreover, the use of a mixture of trifluoroethanol with a second organic solvent even allows the selective production of compound (I) at higher reaction temperatures. Thus, it is possible to prepare highly pure compound (I) in common reactors without special low temperature equipment which is also beneficial for using the process on an industrial scale. Moreover, the compounds of formula (I) are highly soluble in trifluoroethanol so that the reaction volume can be decreased which is a further benefit.

It has been shown that the reaction of compound (II) or a hydrate, solvate or salt thereof can be converted into a compound of formula (I) by any order of addition, i.e. by firstly adding the metal catalyst to the reaction mixture followed by the addition of hydrogen peroxide or a source thereof or alternatively, by firstly adding the hydrogen peroxide or a source thereof to the reaction mixture followed by the addition of the metal catalyst. In a preferred embodiment hydrogen peroxide or a source thereof is added to the dissolved compound of formula (II) or a hydrate, solvate or salt thereof and subsequently the reaction is started by the addition of the metal catalyst.

Preferably, the reaction of step (a) is performed for 1 to 10 hours.

In step (b) of the process according to the invention the produced compound (I) can optionally be recovered from the reaction mixture of step (a). This is preferably effected by one of the following procedures.

In one embodiment, step (b) is performed by at least one of the following steps:
(i) adding acetone or a solution of a thiosulphate salt and optionally a base to the reaction mixture obtained in step (a),
(ii) adding water to the mixture of step (i) to precipitate solid compound (I) and
(iii) separating compound (I).

In step (i) of this recovery procedure acetone or a solution of a thiosulphate salt, preferably sodium thiosulphate dissolved in water, is added to the reaction mixture of step (a), in order to decompose any excess of hydrogen peroxide after the reaction. Moreover, an inorganic base such as sodium hydroxide or potassium hydroxide or an organic base, such as triethylamine, can be added.

In step (ii) water is added to the mixture of step (a) to precipitate compound (I), which can be separated in a conventional manner in step (iii).

In a preferred embodiment of step (iii), the compound (I) obtained in step (ii) is filtered off and optionally recrystallized.

Preferably, recrystallization is performed in a mixture of water and an organic solvent. As organic solvent an alkanol, such as ethanol, 1-methyl-2-pyrrolidone, 1-ethyl-2-pyrrolidone N,N-dimethylacetamide, N,N-dimethylformamide or a mixture thereof can be used. In particular, a mixture of water and 1-methyl-2-pyrrolidone with a ratio of 9:1 to 1:3 (vol/vol) is used. It is also preferred that the recrystallization is carried out in a mixture of water and ethanol in the presence of a weak base, such as triethylamine or ammonia.

Furthermore, in step (iii) the recrystallized compound (I) can be suspended in water, preferably at a temperature of 15 to 20°C and stirred for a certain period of time, such as 2 hours. The obtained product can be collected by filtration and dried, e.g. under reduced pressure at a temperature of 40°C.

In a preferred embodiment, the water used for the suspension of compound (I) has a pH of 8 to 11 which can be adjusted by the addition of a base, such as sodium hydroxide, potassium hydroxide, triethylamine or ammonia. Further, it is preferred that the suspension of compound (I) in water is slowly cooled to 5°C before filtration. It is also preferred that the maceration step is carried out several times, in order to enhance the purity of the compound (I).

According to another embodiment, step (b) is performed by at least one of the following steps:
(i) adding dichloromethane, a solution of a thiosulphate salt and optionally a base to the reaction mixture obtained in step (a),
(ii) removing any solvent of the mixture of step (i) to give crude compound (I),
(iii) adding ethylacetate to crude compound (I) obtained in step (ii) and
(iv) removing the ethylacetate to give compound (I).

In step (i) of this procedure, dichloromethane is added. Further a solution of a thiosulphate salt, preferably sodium thiosulphate dissolved in water, is added to decompose any excess of hydrogen peroxide. Moreover, an inorganic base such as sodium hydrogen carbonate can be added. Afterwards, it is preferred to dry the reaction mixture e.g. by contacting it with sodium sulphate.

In step (ii) the solvent of the reaction mixture of step (i) is removed by e.g. evaporation. Due to complexation reactions with compound (I), it is difficult to completely remove tri-trifluoroethanol. Thus, crude compound (I) is obtained in step (ii). Prior to step (iii), crude compound (I) obtained in step (ii) can be purified, e.g. by passing it through a chromatographic column (for example stationary phase: SiO₂, mobile phase: methanol/dichloromethane 1:9).

Ethylacetate is added to crude compound (I) in step (iii) and the resulting mixture is then concentrated by e.g. evaporation in step (iv). It is preferred that the steps (iii) and (iv) are carried out several times, in order to enhance the purity of compound (I).

Furthermore, step (b) can be carried out by at least one of the following steps:
(i) adding a solution of a thiosulphate salt and optionally a base to the reaction mixture obtained in step (a),
(ii) removing any solvent of the mixture of step (i) to give crude compound (I),
(iii) mixing crude compound (I) obtained in step (ii) with dichloromethane and
(iv) removing the dichloromethane to give compound (I).

According to this procedure, in step (i) a solution of a thiosulphate salt, preferably sodium thiosulphate dissolved in water, is added to the reaction mixture of step (a) to decompose any excess of hydrogen peroxide. Moreover, an inorganic base such as sodium hydrogen carbonate can be added. In step (ii) the solvent of the mixture obtained in step (i) is removed by e.g. evaporating to give crude compound (I). This crude product is in the next step dissolved in dichloromethane which is then removed in step (iv). After step (iv) compound (I) can be recrystallized, for example from ethylacetate.

In case compound (I) is formed as a solid product during step (a), step (b) can be performed by at least one of the following steps:
(i) separating solid reaction product from the reaction mixture obtained in step (a),
(ii) adding dichloromethane to the solid reaction product obtained in step (i) and
(iii) removing the dichloromethane to give compound (I).

In step (i) of this procedure, the solid product obtained in reaction step (a) is separated in a conventional manner from the reaction mixture of step (a). Preferably, the obtained product is filtered off. After separation the product can be further purified. Preferably, it is washed with water.

In step (ii) dichloromethane is added to the product obtained in step (i) to give a solution of compound (I). It is preferred to dry this solution prior to step (iii) by e.g. contacting it with sodium sulphate.

In step (iii) the solvent is removed by e.g. evaporation to give compound (I).

As can be seen from the above specific embodiments, step (b) can generally be performed by reduction of excess hydrogen peroxdie (e.g. by addition of a solution of a thiosulphate salt), addition of an organic solvent such as dichloromethane, drying of the organic phase and removal of the solvent. If no solid product forms, ethylacetate can be added and removed to obtain pure compound (I) in form of a powder.

In step (c) of the process according to the invention the produced compound (I) can optionally be further purified and/or optionally converted into a salt or a solvate or a hydrate thereof.

Generally, compounds of formula (I) such as pantoprazole can be recrystallized using ethylacetate or a mixture of ethylacetate and water.

For example, the purification of compound (I) can be performed by
(i) adding
   (A) ethylacetate or a mixture of ethylacetate and water, and
   (B) optionally a base to the compound (I) obtained in step (b),
(ii) cooling the resulting mixture,
(iii) separating the resulting precipitate of compound (I) and
(iv) optionally washing and drying compound (I) to obtain pure compound (I).

In step (i) of this purification method, ethylacetate or a mixture of ethylacetate and water is added to compound (I) obtained in step (b) of the process according to the invention. If a mixture of ethylacetate and water is used, the ratio between ethylacetate and water can range from 1:100 to 100:1, preferably 10:1 to 1:50, more preferably 1:1 to 1:20 and most preferably is about 1:10, based on the volume of the solvents. In addition to ethylacetate and/or water a base such as sodium hydroxide can be added to compound (I). Preferably, an aqueous solution of sodium hydroxide is used. The molar ratio between compound (I) and sodium hydroxide can range from 200:1 to 1:10, preferably 150:1 to 10:1, more preferably 60:1 to 20:1 and most preferably 40:1 to 30:1.

In step (ii) the resulting mixture is preferably cooled to a temperature ranging from -20 to 25°C, more preferably 0 to 20°C and most preferably 5 to 15°C such as 10 to 15°C to allow compound (I) to precipitate.

In step (iii) the separation of the obtained solid compound (I), e.g. crystalline compound (I), can be performed by methods known in the art such as filtration.

Finally, the separated compound (I) can be washed using for example cooled ethyl acetate and/or subsequently dried to give a pure compound of formula (I).

In another embodiment, compound (I) can be purified by
(i) adding ethylacetate or a mixture of ethylacetate and water to the compound (I) obtained in step (b),
(ii) adjusting the pH to 10 to 15, preferably 11 to 14, more preferably 12 to 14 such as about 13,
(iii) extracting the resulting mixture one or more times with methylene chloride,
(iv) optionally washing the combined organic layers with water,
(v) adjusting the pH of the combined aqueous layers of step (iii) and optionally (iv) to 6 to 9, preferably 7 to 9, more preferably 7.5 to 8.5 such as about 8,
(vi) separating the resulting precipitate of compound (I) and
(vii) optionally washing and drying solid compound (I) to obtain pure compound (I).

In step (i) of this purification method, ethylacetate or a mixture of ethylacetate and water is added to compound (I) obtained in step (b) of the process according to the invention. If a mixture of ethyl acetate and water is used, the ratio between ethyl acetate and water can range from 1:100 to 100:1, preferably 10:1 to 1:50, more preferably 1:1 to 1:20 and most preferably is about 1:10, based on the volume of the solvents.

In the next step (ii), the pH of the resulting mixture is adjusted to 10 to 15, preferably 11 to 14, more preferably 12 to 14 such as about 13. This can for example be effected by adding an aqueous solution of an inorganic base such sodium hydroxide.

In step (iii) the obtained mixture is extracted one or more times with methylene chloride. Preferably, the extraction is performed twice.

After optionally washing the combined organic layers in step (iv), the combined aqueous layers are treated with an acid in step (v) to adjust the pH to 6 to 9, preferably 7 to 9, more preferably 7.5 to 8.5 such as about 8. Preferably, a solution of an inorganic or organic acid such as acetic acid is used. Moreover, it is preferred to cool the combined resulting aqueous layers to a temperature ranging from -20 to 25°C, more preferably 0 to 20 °C and most preferably 5 to 15°C such as 10 to 15°C to allow compound (I) to precipitate.

In step (vi) the separation of the obtained precipitate of compound (I), e.g. crystals of compound (I), can be performed by methods known in the art such as filtration.

Finally, the separated compound (I) can be washed using for example water and/or subsequently dried to give a pure sample of compound (I).

In addition or alternatively to its purification the compound of formula (I) can be converted into a salt or a solvate or a hydrate thereof. For example, pantoprazole obtained according to the process of the invention can be converted into pantoprazole salts such as pantoprazole sodium salt or pantoprazole magnesium salt.

Generally, any forms of compound (II), such as any crystalline forms of pantoprazole sulphide or lansoprazole sulphide, obtained according to any routes of synthesis and prepared via any crystallization methods known in the art can be used in the process according to the invention.

Suitable examples of various crystal forms of pantoprazole sulphide as well as suitable examples of methods for the purification of pantoprazole and the formation of pantoprazole salts such as pantoprazole sodium salt are disclosed in J. Med. Chemistry 1992, 35, 1049-1057, Anal. Profiles of Drug Substances - Pantoprazole Sodium , Vol. 29, 213-259, Chin. Pharm, August 1999, Vol. 34, No. 8, 564-565, IPCOM000016610D of ip.com, Inc., WO 2004/111029, WO 2004/080961, WO 2004/063188, WO 2004/056804, EP 1 300 406, WO 2007/017244, WO 2006/040778, US 2004/0186139, WO 2007/068925, WO 2007/026188, WO 03/097606.

The invention is further illustrated by the following examples.

In the examples, high resolution HPLC was used to determine the purity of compound (I) expressed as area %. The tests were carried out using a Zorbax XDB C18, 1.8 µm, 50 x 4.6 mm. The mobile phase was a gradient of water and water/TEA/acetonitrile (40/0.1/160), pH 7. The chromatograph was equipped with a UV detector set at 285 nm. The flow rate was 0.7 ml/min at 40°C.

### Examples 1 i.-v.:

### Preparation of 5-(difluoromethoxy)-2-[[[3,4-dimethoxy-2-pyridinyl]methyl]sulfinyl]-1H-benzimidazole (pantoprazole) 3.67 g (10 mmol) of 5-difluoromethoxy-2-[[(3,4-dimethoxy-2-pyridinyl]methyl]thio]-1H-benzimidazole (pantoprazole sulfide, anhydrous, crystal form C according to IPCOM000016610D) were dissolved in 10 ml of trifluoroethanol.

Then, 0.908 ml of aqueous H₂O₂ (35%) were added at room temperature to the pantoprazole sulfide solution. Subsequently, the solution was cooled to the temperature specified in the table below and 2.492 mg of CH₃ReO₃ catalyst were added in 2 or 4 portions (intervals of 10 min). The reaction mixture was stirred for one hour at the specified temperature and the reaction was monitored by HPLC analysis.

| Example | Reaction temperature (°C) | No. of portions added |
|---|---|---|
| i. | -10 | 2 |
| ii. | 0 | 2 |
| iii. | -10 | 4 |
| iv. | 0 | 4 |

### Example 2:

### Preparation of 5-(difluoromethoxy)-2-[[[3,4-dimethoxy-2-pyridinyl]methyl]sulfinyl]-1H-benzimidazole (pantoprazole)

### a) Oxidation of pantoprazole sulfide:

1.473 g (4,0 mmol) of 5-(difluoromethoxy)-2-[[[3,4-dimethoxy-2-pyridinyl]methyl]thio]-1*H*-benzimidazole (pantoprazole sulfide, anhydrous, crystal form C according to IPCOM000016610D) were dissolved in 4 mL of trifluoroethanol. 1 mg (0.004 mmol) of CH₃ReO₃ was added and the mixture was stirred at 0°C. 1.5 equiv. of 30% aqueous H₂O₂ (0.60 ml) were added to the cold solution and the reaction mixture was stirred at 0°C for 2 hours.

### b) Recovery of pantoprazole

10 ml of acetone were added to the reaction mixture. Then, the product was precipitated by the addition of 10 ml of water, filtered off, washed with water and dried at 40°C in a vacuum dryer to obtain 1.062 g (72 %) of pantoprazole having a purity of 99.26%; [PN1(pantoprazole sulfide): 0.07%, PN2(pantoprazole sulphone): 0.38%, PN3(pantoprazole sulphone N-oxide): 0.06% and PN4 (pantoprazole N-oxide): 0.04%].

### Example 3:

### Preparation of 5-(difluoromethoxy)-2-[[[3,4-dimethoxy-2-pyridinyl]methyl]sulfinyl]-1H-benzimidazole (pantoprazole)

The oxidation of pantoprazole sulfide described in Example 2 a) was repeated with the exception that 1.469 g of pantoprazole sulfide were used.

20 ml of CH₂Cl₂ were added to the reaction mixture followed by addition of 0.5 ml of a 1M solution of Na₂S₂O₃ to destroy an excess hydrogen peroxide. 0.2 g of solid NaHCO₃ were added and the resulting mixture was stirred for 5 min. Then, Na₂SO₄ was added to dry the reaction mixture. After filtration the solvent of the mixture was evaporated and 2.646 g of crude pantoprazole were obtained. This product was purified by passing it through a short column (2g of SiO₂, methanol/dichloromethane 1:9) to obtain 1.850 g of an oily product. 10 ml of ethylacetate were added to initiate crystallization and evaporated. The addition of 10 ml of ethylacetate and its evaporation was repeated once. 1.550 g (100 %) of crystalline pantoprazole were obtained.

### Example 4:

### Preparation of 5-(difluoromethoxy)-2-[[[3,4-dimethoxy-2-pyridinyl]methyl]sulfinyl]-1H-benzimidazole (pantoprazole)

The oxidation of pantoprazole sulfide described in Example 2 a) was repeated with the exception that 1.471 g of pantoprazole sulfide were used. 0.5 ml of a 1M solution of Na₂S₂O₃ were added to the reaction mixture followed by the addition of 0.2 g of solid NaHCO₃. The solvent was removed under reduced pressure. The crude product was poured into water. However, the product did not precipitate. 20 ml of dichloromethane were added and the aqueous and organic phases were separated. The organic phase was dried by Na₂SO₄ and after evaporation of the solvent 1.956 g of crude pantoprazole were obtained. This product was crystallized from 5 ml of ethylacetate and 761 mg (49 %) of pantoprazole in the form of a white powder were obtained. The remaining product (796 mg, 51 %) was obtained in a pure form after removal of the solvent.

### Example 5:

### Preparation and isolation of 5-(difluoromethoxy)-2-[[[3,4-dimethoxy-2-pyridinyl]methyl]sulfinyl]-1H-benzimidazole (pantoprazole)

The oxidation of pantoprazole sulfide was performed in the same manner as in Example 1, with the exception that at room temperature the CH₃ReO₃ catalyst (2.492 mg) was added to the pantoprazole sulfide solution first, then the reaction mixture was cooled to a temperature of -10°C and finally 35% aqueous H₂O₂ (0.908 ml) was added to the solution. The reaction was carried out for 1 hour at the specified temperature and monitored by HPLC analysis.

At the end of the reaction a solution of 0.56 g of sodium thiosulphate in 4 ml of water was added at a temperature of 5 to 10 °C.

The pH of the obtained mixture was adjusted to 7.5 using aqueous NaOH followed by distillation of trifluoroethanol. 20 ml of a mixture of acetone and water (1:1) were added to the residual mixture which was then stirred for 2 hours at 5 to 10°C. The product was filtered off, washed with water and dried below 45°C. 2.8 g of crystalline pantoprazole were obtained having a purity of 99.23 % (PN2: 0.11%, PN3: 0.05%, PN4: 0.05%).

### Examples 6 i. to v.:

### Preparation of 5-(difluoromethoxy)-2-[[[3,4-dimethoxy-2-pyridinyl]methyl]sulfinyl]-1H-benzimidazole (pantoprazole) 1.473 g (4.0 mmol) of 5-(difluoromethoxy)-2-[[[3,4-dimethoxy-2-pyridinyl]methyl]thio]-1H-benzimidazole (pantoprazole sulfide) were dissolved in 8 ml of a solvent specified in the table below resulting in a 0.5M solution of pantoprazole sulfide. 1 mg (0.004 mmol) of CH₃ReO₃ was added and the mixture was stirred. Then, the reaction temperature was adjusted to the values indicated in the table below and 1.2 equiv. of 30% aqueous H₂O₂ (0.48 ml) were added to the solution. The reaction mixture was stirred at the adjusted temperature for 2 hours (Example 6 i.) or 6 hours (Examples 6 ii. to v.), respectively.

**Table: Preparation of pantoprazole using different solvents**

| **Example** | **solvent** | **reaction conditions** |
|---|---|---|
| i. | CF₃CH₂OH | 0°C, 2h |
| ii. | CH₃CN + CF₃CH₂OH (3:1 vol/vol) | 22°C, 6h |
| iii. | CH₃CH₂OH + CF₃CH₂OH (3:1 vol/vol) | 22°C, 6h |
| iv. | CH₂Cl₂ + CF₃CH₂OH (3:1 vol/vol) | 22°C, 6h |
| v. | *iso*-C₈H₁₈ + CF₃CH₂OH (3:1 vol/vol) | 22°C, 6h |

### Examples 7 i. to vi.:

### Preparation of 5-(difluoromethoxy)-2-[[[3,4-dimethoxy-2-pyridinyl]methyl]sulfinyl]-1H-benzimidazole (pantoprazole) 1.473 g (4.0 mmol) of 5-(difluoromethoxy)-2-[[[3,4-dimethoxy-2-pyridinyl]methyl]thio]-1H-benzimidazole (pantoprazole sulfide) were dissolved in 8 ml of trifluoroethanol resulting in a 0.5M solution of pantoprazole sulfide. Then, a metal catalyst specified in the table below in an amount of 0.1 mol% (corresponds to 0.001 equiv.; Example 7 i.), 1.0 mol% (corresponds to 0.01 equiv.; Examples 7 ii. to 7 v.) or 5.0 mol% (corresponds to 0.05 equiv.; Example 7 vi.) relative to pantoprazole sulfide was added and the mixture was stirred. Subsequently, the reaction mixture was cooled to 0°C and 1.2 equiv. of 30% aqueous H₂O₂ (0.48 ml) were added to the solution. The reaction mixture was stirred at the specified temperature or temperature range (gradient from 0°C to 22°C within the specified time period) The end of the reaction was controlled by TLC.

**Table: Preparation of pantoprazole using different metal catalysts**

| | | |
|---|---|---|
| **Example** | **metal catalyst** | **reaction conditions** |
| i. | CH₃ReO₃ (0.1 mol%) | 0°C, 2h |
| ii. | Na₂MoO₄ (1.0 mol%) | 0-22°C, 2h |
| iii. | V₂O₅ (1.0 mol%) | 0-22°C, 2h |
| iv. | VO(2-OC₃H₇)₃, (1.0 mol%) | 0-22°C, 2h |
| v. | VO(CH₃COCHCOCH₃)₂ (1.0 mol%) | 0-22°C, 2h |
| vi. | VO(CH₃COCHCOCH₃)₂ (5.0 mol%) | 0°C, 4h |

### Example 8: Purification of pantoprazole and formation of the pantoprazole sodium salt.

### i. Purification of pantoprazole

60.0 g of pantoprazole were suspended in 210 ml of ethyl acetate and 0.77 ml of 6 M aqueous sodium hydroxide at a temperature of about 30°C for 0.5 h. Then, the suspension was gradually cooled to 10 to 15°C and stirred for another 2 hours. The product was collected by filtration and washed tree times with 30 ml of cold ethyl acetate. The product was then dried below 45 °C. 53.0 g of crystalline pantoprazole were obtained (99.60% pantoprazole, 0.28% PN2, 0.03% PN3, 0.02% PN4, amount of PN1 below level of detection).

### ii. Purification of pantoprazole

60.0 g of pantoprazole were suspended in 210 ml of aqueous ethyl acetate (10%) and 0.77 ml of 6 M of aqueous sodium hydroxide at about 30°C for 0.5h. Then, the suspension was gradually cooled to 10 to 15°C and stirred for another 2 hours. The product was collected by filtration and washed tree times with 30 ml of cold ethyl acetate. The product was then dried at a temperature below 45 °C. 50.0 g of crystalline pantoprazole were obtained (99.65% pantoprazole, 0.19% PN2, 0.04% PN3, 0.03% PN4, bld PN1).

### iii. Purification of pantoprazole

68.0 g of pantoprazole were suspended in 750 ml of an ethanol/water (1:10) mixture having a pH being adjusted to 13 with a 10 % solution of sodium hydroxide. The reaction mixture was extracted with methylene chloride twice. The combined organic layers were washed with water and the aqueous layer was purified with activated carbon. The pH of the solution was adjusted to 8 ± 0.5 using 17 ml of 50 % acetic acid at about 15 °C to effect crystallization of pantoprazole. The crystallized product was collected by filtration and washed with 50 ml of water. The product was dried at a temperature below 45°C. 61.1 g of crystalline pantoprazole were obtained (99.57% pantoprazole, 0.15% PN2, 0.03% PN3, 0.03% PN4, 0.02% PN1).

### iv. Preparation of pantoprazole sodium sesquihydrate

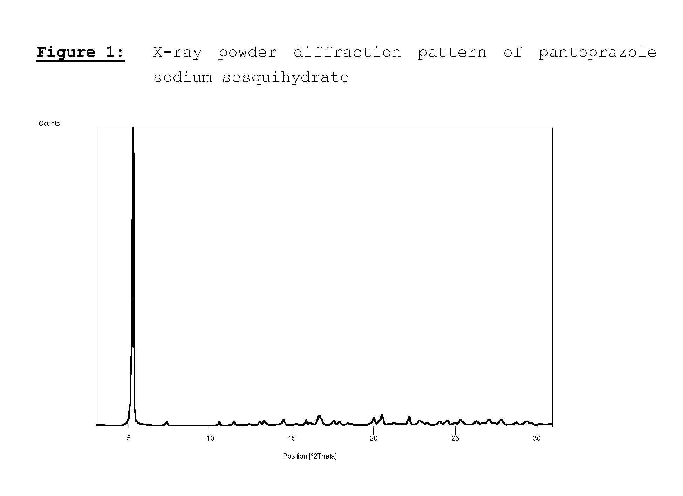

50.0 g of pantoprazole were dissolved in 250 ml of methylene chloride and 15 ml of ethanol at room temperature. The resulting mixture was filtered to remove any undissolved particles. The solution of pantoprazole in methylene chloride and ethanol was treated with 25 ml of 6M aqueous solution of sodium hydroxide until the pH was 12.5 ± 0.5 at 20 ± 3 °C. 500 ml of diisopropyl ether were slowly added to the solution of pantoprazole sodium. Crystallization was carried out at 20 ± 3 °C. The product was collected by filtration and washed with 50 ml of diisopropyl ether. The product was dried in a dryer at a temperature below 45°C. During drying the product was sieved to deagglomerate lumps. 51.5 g of crystalline pantoprazole sodium sesquihydrate were obtained (purity: 99.70%, 0.15% PN2, 0.02% PN3, 0.03% PN4, 0.02% PN1). The X-ray powder diffraction pattern of the obtained pantoprazole sodium sesquihydrate (obtained on a Phillips PW3040/60 X'Pert PRO diffractometer using CuK_{α} radiation, Filter: Ni; Voltage: 45kV; Current: 40mA) is shown in Figure 1. The water content measured according to Ph. Eur. 2.5.12. Method A was about 6.0 to 8.0 wt.-%.

### v. Isolation of pantoprazole magnesium dihydrate

After completion of the oxidation reaction, 5 ml of 5 % sodium thiosulphate solution and 5 ml of water were added at 10 -15 °C to a reaction mixture comprising 4 mmoles of pantoprazole. Then the pH of the resulting mixture was adjusted to 13 using a 10 % solution of sodium hydroxide. The reaction mixture was extracted with methylene chloride twice. The aqueous layer was purified with activated carbon. 2 ml of an aqueous solution of magnesium chloride (2 mmoles) were added to the aqueous layer of pantoprazole sodium with stirring at 20-25°C. After precipitation the solid was filtered, once again suspended in 10 ml of water and stirred for 2 hours. The product was filtered, washed with 5 ml of water and dried at a temperature below 50°C until a water content of 5.0 % (KF) was reached. 1.1 g of pantoprazole magnesium dihydrate were obtained.

### vi. Preparation of pantoprazole sodium sesquihydrate

43.0 g (100 mmoles) pantoprazole magnesium dihydrate were suspended in 400 ml of ethanol at 20-25°C, then 200 ml of water were added and the pH was adjusted to 7.5 - 8.0 using 6 % acetic acid. Pantoprazole was extracted with methylene chloride and clarified with activated charcoal. Collected organic layers were concentrated to 200 ml. The resulting mixture was optionally filtered to remove any undissolved particles. The solution of pantoprazole in methylene chloride and ethanol was treated with 23 ml of a 6M aqueous solution of sodium hydroxide until the pH was 12.5 ± 0.5 at 20 ± 3 °C. 450 ml of diisopropyl ether were slowly added to the solution of pantoprazole sodium. Crystallization was carried out at 20 ± 3 °C. The product was collected by filtration and washed with 50 ml of diisopropyl ether. The product was dried in a dryer at a temperature below 45°C. During drying the product was sieved to deagglomerate lumps. 38.5 g of crystalline pantoprazole sodium sesquihydrate were obtained.

### Example 9:

Preparation of 2-[[[3-methyl-4-(2,2,2-trifluoroethoxy)-2-pyridinyl]methyl]sulfinyl]-1H-benzimidazole (lansoprazole)
a) Oxidation of lansoprazole sulfide: 372 mg (1,0 mmol) of 2-[[[3-methyl-4-(2,2,2-trifluoroethoxy)-2-pyridinyl]methyl]thio]-1*H*-benzimidazole (lansoprazole sulfide) were dissolved in 1 ml of trifluoroethanol. 0.25 mg (0.001 mmol) of CH₃ReO3 were added and the mixture was stirred at 0°C. To the cold solution 1.2 equiv. of 30% aqueous H₂O₂ (0.12 ml) were added and the reaction mixture was stirred at 0°C for 1 hours. Solid product formed during the reaction.
b) Recovery of lansoprazole

The solid product was filtered off and washed with water. 1.51 g of a white solid were obtained which was difficult to dry. The solid was dissolved in 15 ml of dichloromethane. The solution was dried over Na₂SO₄ and the solvent was removed. 361 mg (98 %) of white lansoprazole were obtained.

### Example 10:

Preparation of 2-[[[3-methyl-4-(2,2,2-trifluoroethoxy)-2-pyridinyl]methyl]sulfinyl]-1H-benzimidazole (lansoprazole)

The oxidation of lansoprazole sulfide described in Example 9 a) was repeated.

10 ml of dichloromethane were added to the reaction mixture, followed by the addition of 0.3 ml of a 1M solution of Na₂S₂O₃. The resulting mixture was dried over Na₂SO₄ and the solvent was removed. 666 mg of an oily product were obtained. 10 ml of ethylacetate were added to the product and the mixture was concentrated by evaporation. 371 mg (100%) of lansoprazole having a purity of 97.56% were obtained.

### Example 11:

Purification of lansoprazole
i.) 48 g of wet lansoprazole were dissolved in 80 ml of 1-methyl-2-pyrrolidone (NMP) and 5 ml of triethylamine, cooled to 15 °C and the product was precipitated with 240 ml of water. The suspension was stirred for 4-10 hours and filtered to obtain 34.9 g wet lansoprazole . The wet crystallized product was suspended in 200 ml of water treated with triethylamine so that the pH value was adjusted to about 9-10. The suspension was cooled down to 15°C, stirred at a temperature of from 15 to 20°C for 2 hours and then cooled to a temperature of from 0 to 5°C. The obtained crystals were collected by centrifugal filtration and dried under reduced pressure at 40°C to obtain 26.6 g of lansoprazole form A.
ii.) Example 11 i. was repeated with the exception that the pH of the suspension of wet lansoprazole was adjusted by sodium hydroxide instead of triethylamine.
iii.) Example 11 i. was repeated with the exception that wet crystallized lansoprazole was suspended in potable water instead of water having a pH of 9 to 10.
iv.) 48 g wet lansoprazole were dissolved in a mixture of 110 ml of 1-methyl-2-pyrrolidone (NMP), 2.67 g of sodium hydroxide and 75 ml i-propyl acetate at 20 ± 2 °C . The resulting layers were separated, the aqueous phase was cooled to 10°C and the product was precipitated by addition of 8.3 ml of 50 % acetic acid to give a pH value between 9.8 and 10.1 at 5-10°C . The suspension was stirred for two hours at 5-10°C, then filtered, and washed with 20 ml of water to obtain 36.8 g of wet lansoprazole. The wet crystallized product was suspended in 228 ml of water having a pH value of about 9-10 (treated with sodium hydroxide) at 16 to 18°C, stirred for two hours and cooled to 5°C . The product was filtered, washed with 10 ml of water and dried under reduced pressure at 40°C to obtain 26.7 g of lansoprazole form A.
v.) Example 11 iv. was repeated with the exception that formic acid was used instead of acetic acid.
vi.) Example 11 iv. was repeated with the exception that hydrochloric acid was used instead of acetic acid.
vii.) 1.58 kg of wet crude lansoprazole were suspended in an ethanol/water/ammonia mixture (5.2 : 0.6 : 0.006. 1) and the mixture was heated to 50°C. The insoluble material was filtered off, the filtrate was gradually cooled and when the crystallization started (32°C), the suspension was vigorously stirred and cooled to 0°C. The suspension was stirred for an additional 0.5 hours. The solid was filtered, washed with 1.0 1 of a cold ethanol/water mixture (9 : 1) to obtain 1.19 kg of wet product which is suspended at 22°C in 6.65 1 of water with a pH of 9.66 adjusted with triethylamine. The suspension was stirred for two hours at 20 ± 2 °C, then cooled in one hour to 5°C, filtered and washed with 1.0 1 of water with a pH of 9.0 to obtain 1.55 kg of wet product. Maceration in water was once again repeated. The wet product (1.48 kg) was dried in vacuo at 40°C to give 0.71 kg of lansoprazole form A having a BET-surface area of 4.67 m²/g.

## Claims

1. Process for preparing a compound of formula (I) 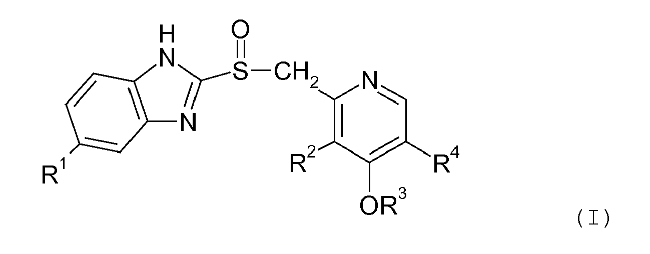 or a salt or a solvate or a hydrate thereof, wherein
R¹ is selected from the group consisting of hydrogen, C₁ C₄-alkyl and C₁-C₄-alkoxy, wherein the C₁-C₄-alkyl and C₁-C₄-alkoxy are unsubstituted or substituted with one or more halogen,
R² is selected from the group consisting of hydrogen, C₁ C₄-alkyl and C₁-C₄-alkoxy, wherein the C₁-C₄-alkyl and C₁-C₄-alkoxy are unsubstituted or substituted with one or more halogen,
R³ is C₁-C₄-alkyl which is unsubstituted or substituted with one or more halogen or one or more C₁-C₄-alkoxy, and
R⁴ is selected from the group consisting of hydrogen and C₁-C₄-alkyl which is unsubstituted or substituted with one or more halogen, comprising
(a) oxidizing a compound of formula (II) 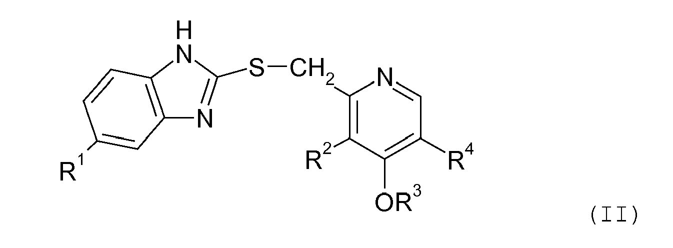 or a hydrate, solvate or salt thereof, in which R¹, R², R³ and R⁴ have the same meanings as defined for compound (I), to give a compound of formula (I), wherein the oxidizing is performed in the presence of trifluoroethanol,
(b) optionally recovering the compound of formula (I) and
(c) optionally purifying the compound of formula (I) and/or converting it into a salt or a solvate or a hydrate thereof.

2. Process according to claim 1, wherein R¹ is difluoromethoxy, R² is methoxy, R³ is methyl and R⁴ is hydrogen.

3. Process according to claim 1, wherein R¹ is hydrogen, R² is methyl, R³ is 2-trifluoroethyl and R⁴ is hydrogen.

4. Process according to claim 1, wherein R¹ is methoxy, R² is methyl, R³ is methyl and R⁴ is methyl.

5. Process according to claim 1, wherein R¹ is hydrogen, R² is methyl, R³ is 3-methoxypropyl and R⁴ is hydrogen.

6. Process according to any one of claims 1 to 5, wherein the oxidation is performed in the presence of hydrogen peroxide or a source thereof.

7. Process according to claim 6, wherein the hydrogen peroxide is used in the form of an aqueous solution of hydrogen peroxide.

8. Process according to claims 6 or 7, wherein the source of hydrogen peroxide is a urea adduct of hydrogen peroxide.

9. Process according to any one of claims 6 to 8, wherein the hydrogen peroxide is present in an amount of 0.5 to 3.0 equivalents, preferably 0.7 to 2.0 equivalents and most preferably 0.9 to 1.5 equivalents relative to compound (II).

10. Process according to any one of claims 1 to 9, wherein the oxidation is performed in the presence of a metal catalyst.

11. Process according to claim 10, wherein the metal of the metal catalyst is selected from the group consisting of rhenium, vanadium, molybdenum, tungsten, cerium and ytterbium.

12. Process according to claim 11, wherein the metal catalyst is selected from the group consisting of CH₃ReO₃, C₂H₅ReO₃, Re(PPh₃)₂OCl₃, Na₂MoO₄, V₂O₅, VOCl₃, VOF₃, VO(OC₂H₅)₃, VO(1-OC₃H₇)₃, VO (2-OC₃H₇)₃, VO (CH₃COCHCOCH₃)₂, NaVO₃, H₂WO₄, H₄SiW₁₂O₄₀, (NH₄)₂Ce(NO₃)₆ and Yb(OSO₂CF₃)₃.

13. Process according to claim 11 or 12, wherein the metal catalyst is present in an amount of 0.0001 to 0.1 equivalents, preferably 0.0002 to 0.01 equivalents and most preferably 0.0005 to 0.0015 equivalents relative to compound (II).

14. Process according to any one of claims 1 to 13, wherein step (a) is performed in the presence of trifluoroethanol and an organic solvent.

15. Process according to claim 14, wherein said organic solvent is selected from the group consisting of methanol, ethanol, acetone, acetonitrile, C₆H₅CF₃, ethers such as THF, unpolar solvents such as dichloromethane and *iso-*alkanes, and mixtures thereof.

16. Process according to any one of claims 1 to 15, wherein compound (II) is employed in step (a) in a concentration of 0.1 to 5 mol/l, preferably 0.2 to 2 mol/l.

17. Process according to any one of claims 1 to 16, wherein step (a) is carried out at a temperature ranging from -30 to 30°C, preferably from -10 to 30°C.

18. Process according to any one of claims 10 to 17, wherein hydrogen peroxide or a source thereof is added to the dissolved compound of formula (II) or a hydrate, solvate or salt thereof and subsequently the reaction is started by addition of the metal catalyst.

19. Process according to any one of claims 1 to 18, wherein step (b) is performed by at least one of the following steps:
(i) adding acetone or a solution of a thiosulphate salt and optionally a base to the reaction mixture obtained in step (a),
(ii) adding water to the mixture of step (i) to precipitate solid compound (I) and
(iii) separating compound (I).

20. Process according to any one of claims 1 to 18, wherein step (b) is performed by at least one of the following steps:
(i) adding dichloromethane, a solution of a thiosulphate salt and optionally a base to the reaction mixture obtained in step (a),
(ii) removing any solvent of the mixture of step (i) to give crude compound (I),
(iii) adding ethylacetate to crude compound (I) obtained in step (ii) and
(iv) removing the ethylacetate to give compound (I).

21. Process according to any one of claims 1 to 18, wherein step (b) is performed by at least one of the following steps:
(i) adding a solution of a thiosulphate salt and optionally a base to the reaction mixture obtained in step (a),
(ii) removing any solvent of the mixture of step (i) to give crude compound (I),
(iii) mixing crude compound (I) obtained in step (ii) with dichloromethane and
(iv) removing the dichloromethane to give compound (I).

22. Process according to any one of claims 1 to 18, wherein step (b) is performed by at least one of the following steps:
(i) separating solid reaction product from the reaction mixture obtained in step (a),
(ii) adding dichloromethane to the solid reaction product obtained in step (i) and
(iii) removing the dichloromethane to give compound (I).
